# EUROPEAN PATENT SPECIFICATION

(11) **EP 0 397 256 B1**
(45) Date of publication and mention of the grant of the patent: **02.03.1994**
(21) Application number: 90201133.7
(22) Date of filing: 04.05.1990
(51) Int. Cl.: G01N 35/00

(54) **Analyser featuring a circular track of cartridges centered on an incubator and method of use**
Analysator mit kreisförmiger, auf einen Inkubator zentrierter Kassettenbahn und Verfahren zu seiner Verwendung
Analyseur avec piste circulaire de cassettes au tour d'un incubateur, et méthode d'utilisation

(30) Priority: 09.05.1989 US 349451; 28.03.1990 US 500651
(43) Date of publication of application: 14.11.1990
(73) Proprietor: EASTMAN KODAK COMPANY, Rochester, New York 14650-2201 (US)
(72) Inventor: Shaw, James David, C/o Eastman Kodak Company, Rochester, New York 14650 (US); Muszak, Frank Martin, c/o Eastman Kodak Company, Rochester, New York 14650 (US)
(74) Representative: Mackett, Margaret Dawn

(56) References cited:
- US-A- 4 512 952
- PATENT ABSTRACTS OF JAPAN, vol. 7, no. 278 (P-242)[1423], 10th December 1983; & JP-A-58 156 833
- PATENT ABSTRACTS OF JAPAN, vol. 7, no. 90 (P-191)[1235], 14th April 1983; & JP-A-58 015 157

## Description

This invention relates to an analyzer and a method for the economical assaying of components of a body liquid, particularly designed for high volume throughput occupying a minimum amount of space.

Large liquid analyzers such as the blood analyzers described in USA-4512952 have been very successful in testing body liquids using dried test elements sometimes identified as "slides". One of the reasons for this has been the high throughput which such analyzers provide, and the high degree of accuracy and precision which is possible. On the other hand, there remain some minor disadvantages. These stem from the fact that three different kinds of slide test elements have been used in such analyzers, namely, colorimetric end-point types (hereinafter "CM" types); rate types; and potentiometric types (hereinafter "PM" types). Each type has needed its own incubation and reading station. Examples are disclosed in US-A4512952. Due to the need for three incubators and their separate read stations, such analyzers have taken up considerable space. Furthermore, triplicating the incubators and read stations has added considerably to the cost.

A further disadvantage of some analyzers has been the amount of space required. Every time an additional function is added, the apparatus for that function has been simply "added on", producing a machine wherein no attempt is made to use space most efficiently.

Japanese Kokai 61/209341 describes a rotating incubator which is said to be useful for CM type test elements or rate-type elements. However, PM types are not mentioned, nor are the test elements supplied from sources which provide for maximum utilization of space in the analyzer.

Therefore, prior to this invention, there has been a problem in providing for a blood analyzer having high throughput, accuracy and precision as in the above-noted prior analyzer, but which uses fewer stations in a more efficient manner to require less room and less cost.

It is therefore an object of the present invention to provide an analyzer which allows all three of the above-mentioned types of dried test elements to be assayed on one incubator, and which solves the above-noted problems.

More specifically, in accordance with one aspect of the present invention, there is provided an analyzer for assaying for analytes of a body liquid on dried test elements, the analyzer including:-
means for temporarily storing a stack of dried test elements, the test elements in any one stack being for the same assay and are selected from any one of potentiometric, colorimetric or rate-type test elements;
an incubator for incubating test elements taken from a stack; and
detection means for detecting a change in a test element after incubation in the incubator;
characterized in that the analyzer further includes at least one generally circular horizontal path centered generally on the incubator and surrounding said incubator;
moving means for moving the stack-storing means around each path;
holding means for temporarily holding the stack-storing means on the moving means, the holding means being movable between at least two positions; and
transfer means for transferring a test element to the incubator from any stack-storing means held on a circular path.

In accordance with a second aspect of the present invention, there is provided apparatus for temporarily supporting a stack of planar slide-like elements, in a cartridge held in a predetermined vertical direction, the apparatus comprising
means for temporarily holding a cartridge of elements,
means for moving the holding means along a generally circular path around a center,
characterized in that the apparatus includes means for slidably moving the holding means between two positions on a radius of the circular path, one of the positions being one which allows a cartridge to fall, and the other of the positions being one which holds up a cartridge.

In accordance with a third aspect of the present invention, there is provided a method for supplying a test element to an incubator, each test element being selected from any one of three different types of test elements, the method comprising the steps of:-
supplying slide-like test elements as a plurality of stacks, the test elements of each stack being identical within the stack,
mounting the stacks on at least one generally circular path surrounding a central incubator,
moving the stacks around the path until a selected stack reaches a predetermined position,
and transferring a test element from the stack at the predetermined position to the incubator.

It is an advantageous feature of the invention that an analyzer for all three types of test elements is provided which minimizes the space needed, without sacrificing through-put.

It is a related advantageous feature that such an analyzer is laid-out in a highly efficient configuration which requires less space.

It is another advantageous feature of the invention that apparatus and a method are provided for efficiently and easily loading any one of three different kinds of test elements into a single incubator.

The present invention will now be described by way of example only with reference to the accompanying drawings in which:
Figure 1 is a perspective view of an analyzer constructed generally in accordance with the invention;
Figure 2 is a partially schematic, plan view depicting the general arrangement of the parts of the analyzer, the cover and rotor of the incubator having been removed for clarity;
Figure 3 is a fragmentary perspective view of a portion of the cartridge-mounting means according to the present invention, nesting frames having been removed for clarity;
Figure 4 is a fragmentary exploded sectioned view taken generally along the line IV-IV of Figure 3, with the nesting frames added;
Figure 5A is fragmentary sectioned view taken generally along the line VA-VA of Figure 4;
Figure 5B is a fragmentary sectioned view taken generally along the line VB-VB of Figure 4;
Figure 6 is a sectioned view taken generally along the line VI-VI of Figure 5;
Figure 7 is an exploded sectioned view taken generally along the line VII-VII of Figure 6, but without the spring (for clarity);
Figure 8 is a fragmentary sectioned elevational view taken from behind transfer device 400 as seen in Figure 1, illustrating the cartridge-mounting means assembled in place to cooperate with a pusher blade of device 400 to dispense test elements to the incubator;
Figure 9 is a fragmentary sectioned elevational view similar to that shown in Figure 8, but illustrating the parts in the cartridge-release position;
Figure 10 is a fragmentary sectioned elevational view similar to a portion shown in Figure 8, but illustrating an alternate embodiment;
Figure 11 is a fragmentary plan view taken generally along the line XI-XI of Figure 10;
Figure 12 is a fragmentary elevational view similar to that shown in Figure 10, but of yet another alternate embodiment;
Figure 13 is a fragmentary sectioned elevational view of a mechanism for loading cartridges into the mounting means shown in Figure 8;
Figure 14 is a fragmentary sectioned elevational view of a pusher blade, housing and drive means useful with this invention;
Figure 15 is a fragmentary sectioned elevational view of an incubator useful in this invention;
Figure 16 is a fragmentary plan view looking up, of a mechanism for unloading cartridges from the mounting means shown in Figure 8, using the doors partially shown in Figure 10; and
Figure 17 is a schematic illustration of a preferred reflectometer useful in the invention.

The invention is hereinafter described in detail with respect to preferred embodiments featuring certain kinds of dried test elements, and preferred specific mechanisms a) for supplying and dispensing body liquid for assaying, b) for unloading test elements from cartridges, and c) for incubating such test elements after body liquids are dispensed thereon. In addition, the invention is useful regardless of the chemistries used in the dried test elements, and the type of cartridge, and regardless of the body liquid assayed or the specific mechanisms contributing the noted functions of supplying and dispensing body liquid, and unloading and incubating test elements.

Orientations such as "up", "horizontal", "level" and the like are used herein with respect to the orientations preferred during normal usage.

The preferred test elements for use in this invention are the dried test elements disclosed in patents such as US-A-4336091 (PM-type); and US-A-3992158 and US-A-4258001 (for both CM-type and rate type). Useful elements also include all three noted types as they are available under the trademark "Ektachem" slides from Eastman Kodak Company.

The test elements are provided as stacks within the storage means described below, each stack preferably comprising identical test elements for a single assay. Any method of providing such stacks is useful. Preferably, each stack is housed in a cartridge, the term hereinafter used. Most preferably, such cartridges are constructed as described in any of re-issued US Patent 30,595; US-A-4187077; US-A-4151931; and US-A-4190420. Additionally, the cartridges available under the trademark "Ektachem" slide cartridges for specific assays available from Eastman Kodak Company can be used.

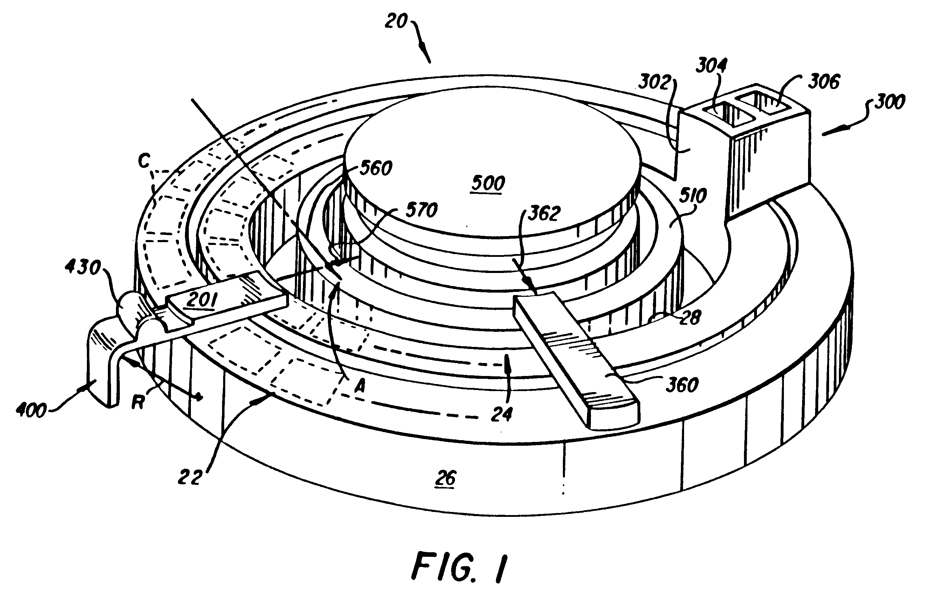

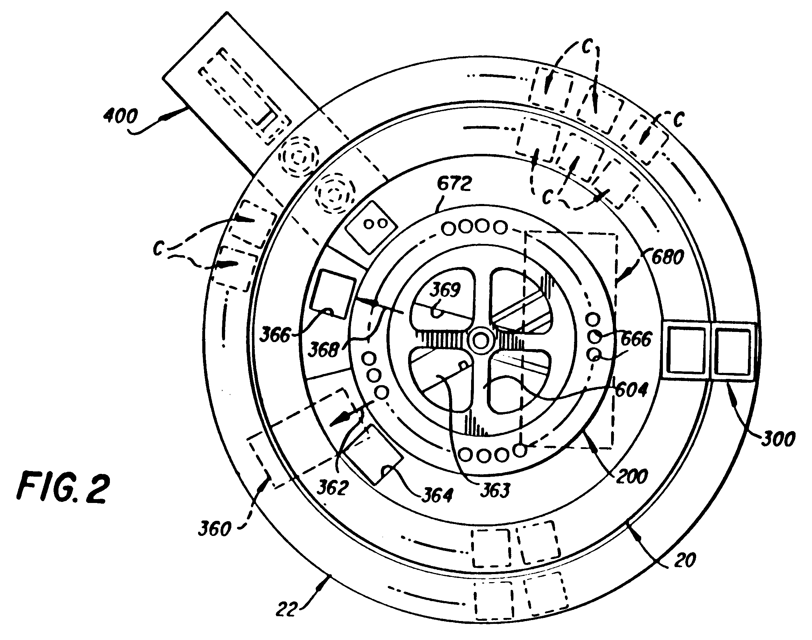

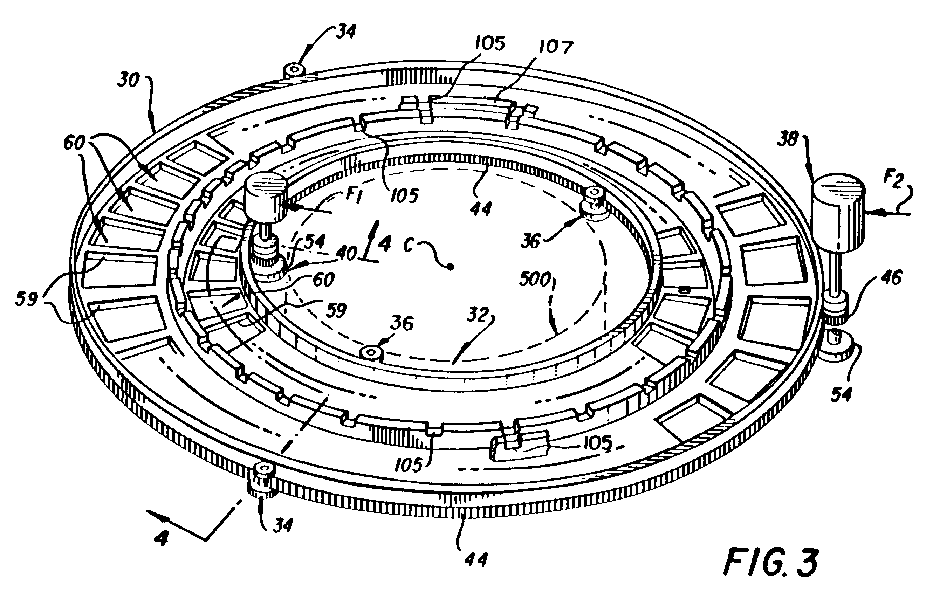

In accordance with the invention, as shown in Figures 1 and 2, analyzer 20 comprises means for temporarily storing a plurality of stacks of test elements are provided in the form of cartridges C. Means are further provided to define at least one, and preferably two, concentrically disposed paths 22 and 24, preferably centered on point c, the center of the incubator and of the analyzer as shown in Figure 3. Each path 22, 24 extends around a single incubator 500 which is disposed so as to be generally centered within the paths, thus optimizing the use of space in the analyzer. A cartridge-loading station 300, as shown in Figures 1 and 2, is disposed above the paths to one side of incubator 500. An electrometer 360 is disposed above the paths at another side of incubator 500, and a test-element transfer device 400 is provided above the paths at yet another side of incubator 500 to load a test element into incubator 500 from any cartridge on the paths. A stationary support surface 510 is provided as an annular ring between path 24 and incubator 500, to cooperate with a liquid-dispensing probe, described hereinafter. The analyzer controls, not shown, comprise one or more microprocessors connected to suitable input and output devices, as is conventional.

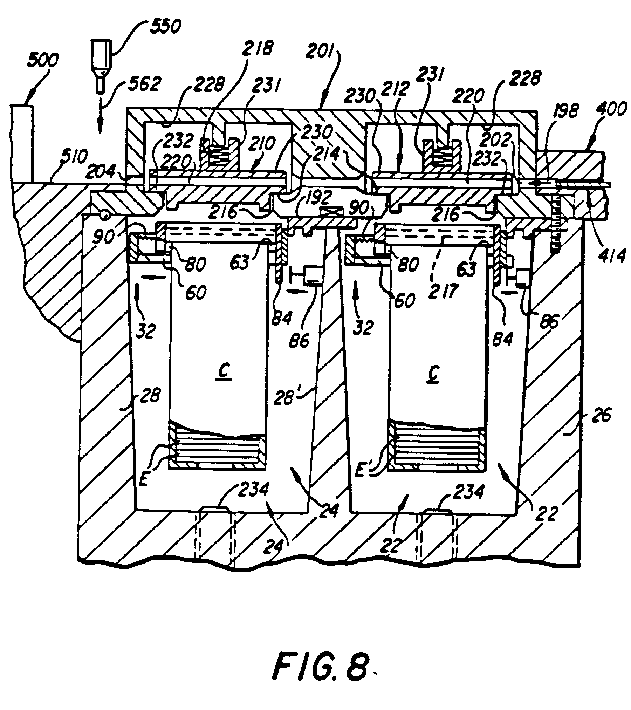

Referring more specifically to paths 22 and 24, each of these is separately housed within a frame comprising fixed exterior walls 26 and 28, respectively, as shown in Figures 1 and 8. Most preferably, walls 26 and 28 can be combined to provide a single common wall 28' between the paths, see Figure 8.

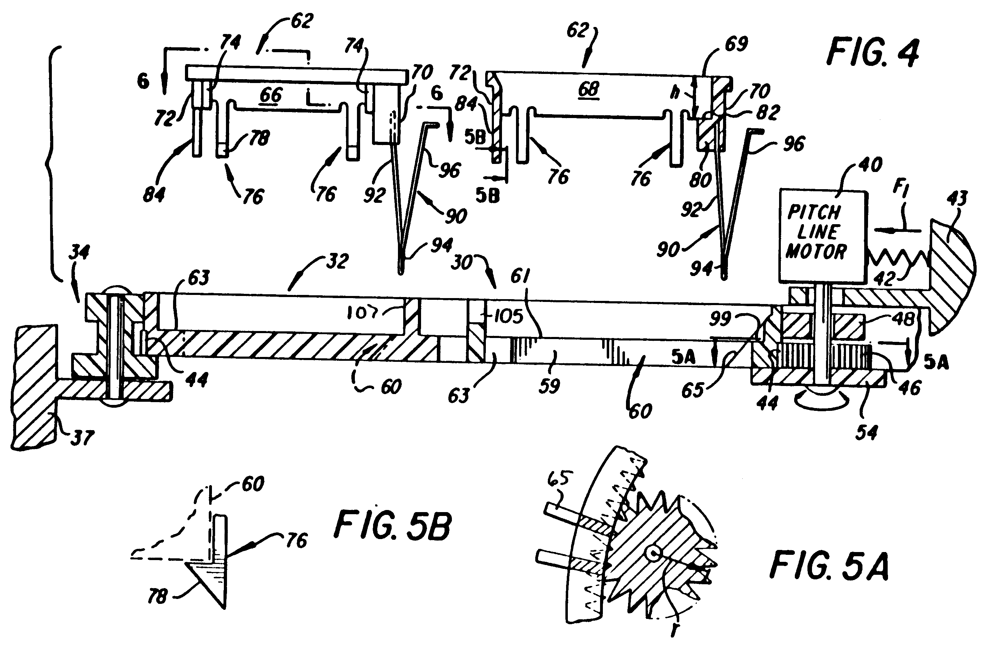

To move cartridges around each path, within each housing for each path, an annular ring 30 or 32 is rotatably mounted, as shown in Figures 3 to 5 and 8. Follower rollers 34, 36 are strategically placed on frame portion 37 of the analyzer, as shown in Figure 4, to partially constrain the rings on the horizontal and vertical directions. The final constraint is a drive motor 38, 40 as shown in Figures 3 and 4. Each drive motor is biased by a conventional spring 42 (Figure 4) mounted on a frame member 43 of the analyzer to exert a radial force F₂ or F₁ against the ring, see Figures 3 and 4 respectively. The drive connection is via a ring gear 44 mounted on the outside of ring 30 and the inside of ring 32, and a pinion gear 46 on the drive motor. Motors 38 and 40 are preferably conventional pitch-line motors, that is, in addition to the pinion gear 46, there is a smooth roller 48 mounted integrally with and above the pinion gear 46, and the radius r (Figure 5A) of this roller is adjusted to ensure that teeth 50 of the pinion gear always engage teeth 52 of the ring gear on the pitch line. Additionally, each motor 38, 40 preferably includes a rotating disk 54 which provides underlying vertical support of the ring (Figures 3 and 4).

Each of the rings 30 and 32 has cut-out portions 60 shaped and sized to accommodate a test-element cartridge, as shown in Figure 8. In ring 30, thirty-five such cut-outs are preferably placed, with a fewer number in ring 32 (Figure 3).

In accordance with another aspect of the invention, holding means are included with each of paths 22 and 24, and with each ring 30 and 32, to temporarily hold a cartridge on the ring, preferably in a vertical orientation. The holding means are constructed to operate between at least two alternate states; one of which holds the cartridges as noted, and the other of which releases the cartridge from the ring, preferably to fall away from the ring.

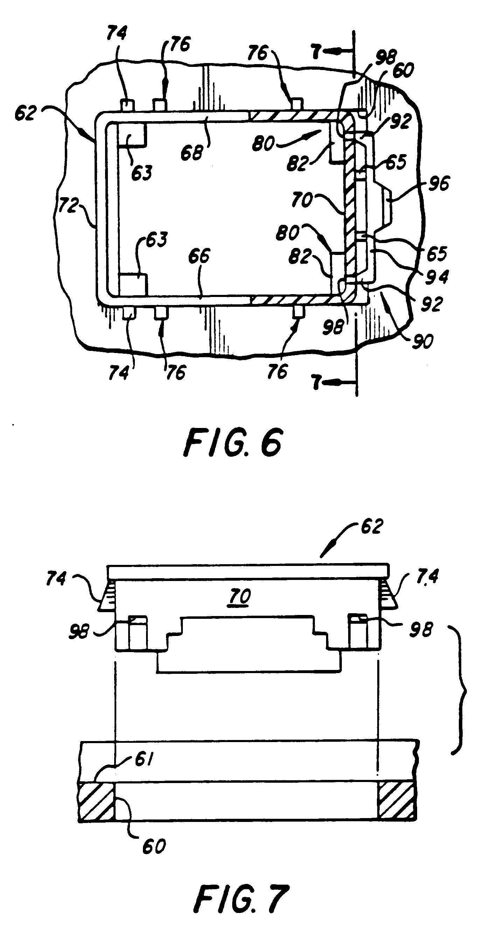

Any such holding means can be used. Highly preferred is one which cooperates with the upper portion of each ring and which moves with respect thereto to release a cartridge. More specifically, an upper, horizontal support surface 61 is provided on each ring around each cut-out portion (Figure 4). Sidewall 59 of each cut-out 60 is generally oriented radially with respect to the center of ring 30 or 32 (Figure 3). As seen in detail in plan in Figure 6, each cut-out portion includes at the outer end, a pair of shoulders 63 projecting out into the overall area normally occupied by an upper end of a cartridge (Figure 8). At the inner end of the cut-out, two diagonally shaped guide flanges 65 are provided (see Figures 4 and 6). Shoulders 63 thus act as means for holding vertically a cartridge, when such shoulders are correctly spaced with respect to a nesting frame member discussed hereinafter.

Most preferably, the cartridges are temporarily held in each ring by "hanging" them in the cut-out portions (Figure 8). A preferred form of such holding or "hanging" means comprises nesting frame members 62 for each cut-out portion, as shown more clearly in Figures 4 and 6 to 8, which cooperate with shoulders 63. More specifically, each such nesting member 62 comprises a generally rectangular frame having two generally vertical side walls 66,68 and two generally vertical end walls 70, 72 (Figure 4). Each side wall has preferably two projections 74, see in particular Figures 4, 6 and 7, which serve to ride on the upper supporting surface 61 of the ring to keep frame member 62 from falling through cut-out portions 60. In addition, each side wall preferably includes two spring fingers 76 which have a latch shoulder 78 (Figure 5B). The fingers are biased outwardly to releasably hold frame member 62 within cut-out portions 60.

To provide part of the means vertically supporting a cartridge, end wall 70 includes at least one and preferably a pair of cartridge support shoulders 80 with a supporting surface 82, as shown in Figure 6. Surface 82 is positioned below top surface 64 of frame member 62 at a distance h (Figure 4) which equals the distance from top surface 64 to support surface 61 of rings 30 and 32. Shoulder 80 and surface 82 thus project into cut-out portion 60 at generally the same horizontal level as surface 61. When the opposite end wall 72 is pushed over the exposed shoulder 63 of surface 61, surface 82 of nest frame 62 is thus aligned and co-acts with surface 61 of shoulder 63 to hold up a cartridge, as shown in Figure 8.

Opposite end wall 72 includes a downwardly-depending pusher lip 84, as is shown more clearly in Figures 4, 7 and 8, which cooperates with nest moving means 86 mounted on common wall 28' and wall 26 of the paths. The means 86 can be a solenoid (as shown) or a motor-actuated lever, or any automatic conventional actuator.

To bias the nesting frame into the "normal" or "hanging" position shown in Figure 8, spring means are mounted from end wall 70 (Figure 4). Most preferably, the spring means comprise a compression leaf spring 90 having two interior legs 92 (Figure 6) connected to a body portion 94 from which protrudes upwardly a middle leg 96 (Figure 4). The ends of legs 92 engage apertures 98 of end wall 70 (Figures 6 and 7) whereas the end of leg 96 rests on surface 61 at position 99 between the two guide flanges 65, Figure 4.

Alternatively, spring 90 can be an integral part of frame 62.

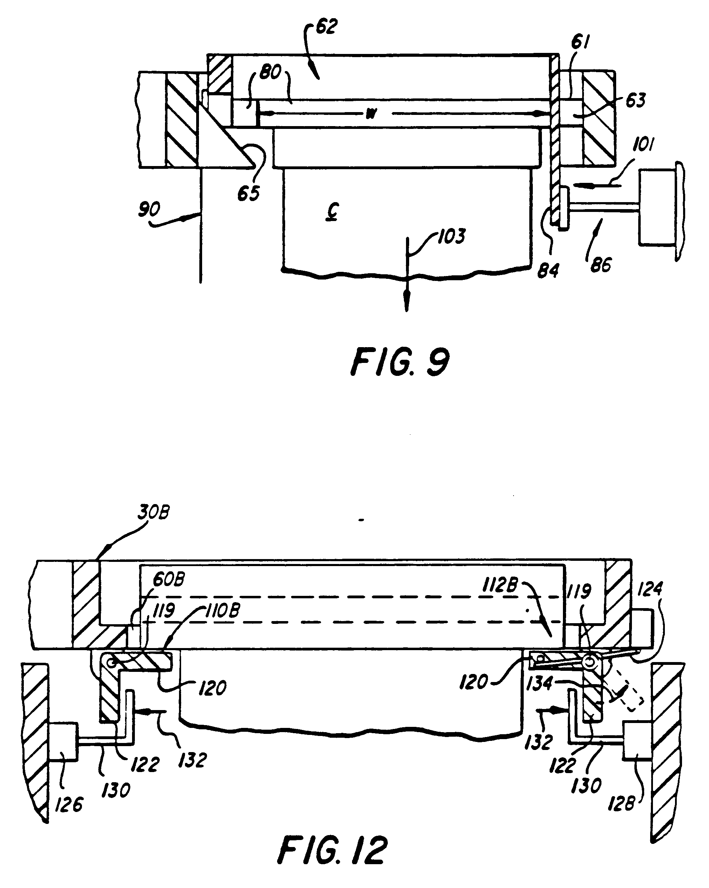

In use, spring 90 (shown schematically in Figure 8) "normally" biases nesting frame 62 towards the outside of the ring, generally along a radius of the ring, so that one edge of cartridge C is supported or "hung" by shoulder surface 63 of the ring, and the other is supported by shoulder 80 of nesting frame 62. The exception occurs when override means 86 is actuated in either path 22 or 24. When that occurs, nesting frame 62 is moved leftward to compress spring 90 as indicated by arrow 101 in Figure 9. The upper lip of cartridge C is pushed off surface 61 at lip 63 by leftward-moving lip 84 of nesting frame 62. As the frame 62 continues to move leftward, the opening between oppositely-paired shoulders 80 and 63 gradually increases until the spacing w is sufficient to allow cartridge C to fall through, in the direction indicated by arrow 103. Complete exiting of this cartridge from the ring requires, however, the actuation of an exit door 326, discussed hereinafter.

Alternatively, (not shown), cut-outs 60 and frame members 62 could be rotated 90° so as to slide relative to each other (to hold or release a cartridge) along an arc, that is, along a portion of the path traversed by the ring carrying the frame.

Notches 105 are preferably included on the outside flange edge of ring 32, and the inside flange edge 107 of ring 30 (Figure 3) to allow a sensor (not shown) to sense that a particular cartridge is in fact at a particular location, e.g., the cartridge load/unload station 300. Most preferably, each notch is aligned with the middle of the corresponding cut-out portion 60 (Figure 4) although other locations are also useful. (Only a portion of flange edge 107 is shown in Figure 3.) Each of the cut-out portions 60 can be tracked by the analyzer by flagging one of them as a home position, as is conventional, and then counting the number of offsets from "home".

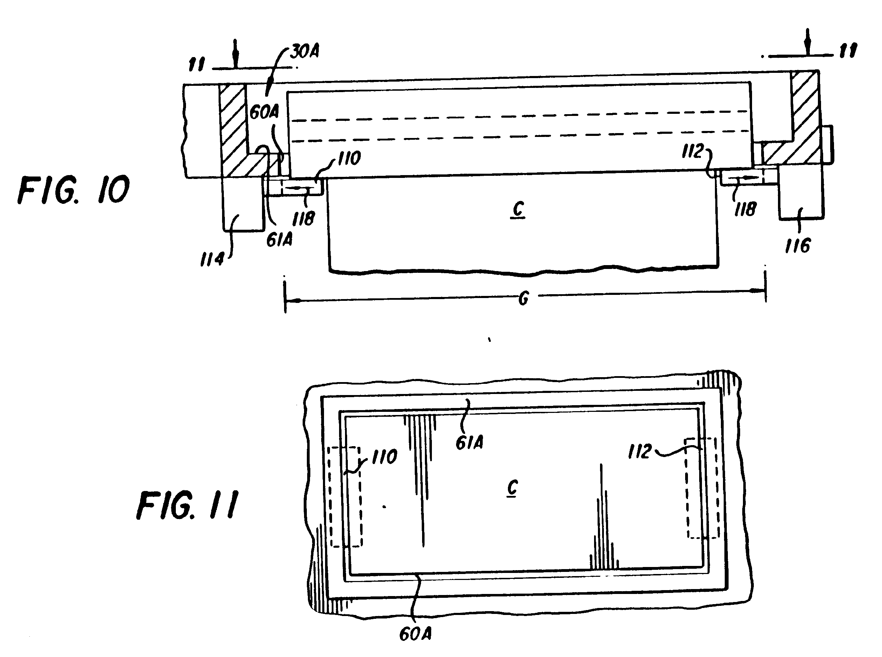

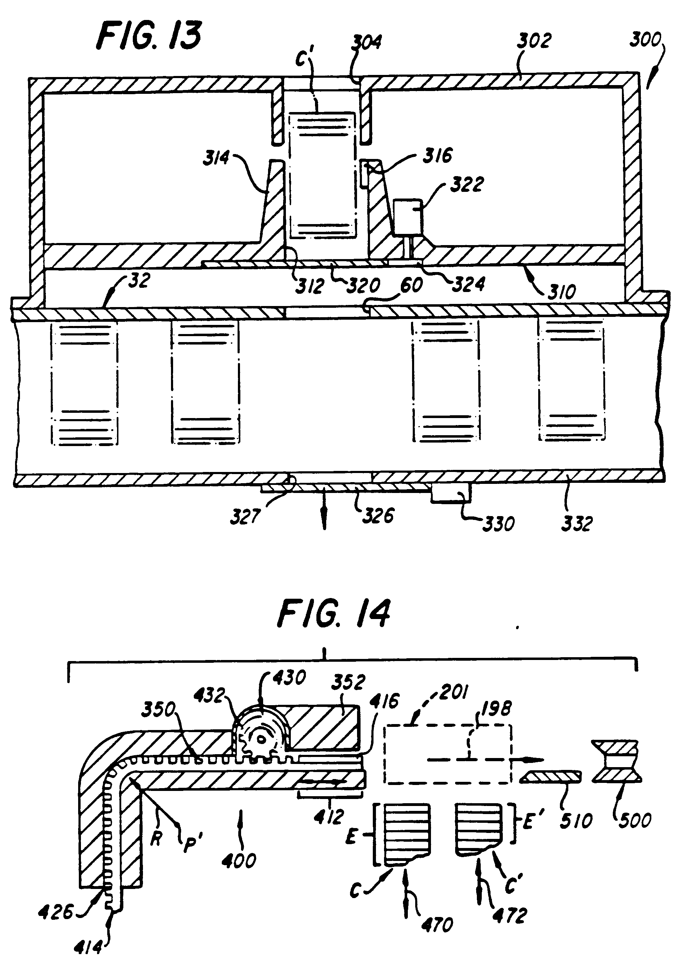

Still further, the holding member for the cartridges need not be a full frame which sits and moves within cut-outs in the ring. Instead, as shown in Figures 10 and 11, it can be simply solenoid-actuated fingers which hold up the cartridge. Parts similar to those previously described bear the same reference numeral, to which the distinguishing suffix A is appended.

Thus, each ring such as ring 30A can have cut-out portions 60A, which are larger in their interior dimensions (see Figure 11) than the outside dimensions of the upper end of cartridge C. As a result, none of surface 61A surrounding the cut-out portions 60A is used to support the cartridge. Indeed, except for the fingers hereinafter described, cartridge C would fall right through the opening of portion 60A. In this embodiment, the cartridge-holding means comprise oppositely disposed fingers 110, 112, actuated by solenoids 114, 116. When in the solid position shown in Figure 10, the fingers are extended and hold up a cartridge. When withdrawn to the dotted position, as indicated by arrow 118, they are separated too far apart to hold a cartridge, so that gap G causes the cartridge to fall through. Alternatively, not shown, one of fingers 110 and 112 can be fixed in the extended position and the other withdrawable a sufficient distance as to create the gap G.

Such solenoid-activatable fingers require a brush-less connection between the rotating ring and a power source. Alternatively, the fingers 110 and 112 can be pivoted so as to be cammable between two positions by a solenoid mounted on the stationary frame. Parts similar to those previously described bear the same reference numeral, to which the distinguishing suffix B is appended.

Thus, ring 30B has a cut-out portion 60B, Figure 12, through which a cartridge C will fall, as in Figure 10, except for opposed fingers 110B and 112B which are pivotably mounted at 119 to move between two alternate states. In one state, as shown in solid lines, they project into the path of the cartridge to catch and hold the cartridge. The fingers are right-angled, with one end 120 locatable under the cartridge and opposite end 122 positioned 90° around and below pivot 119. Spring biasing means such as a compression spring 124, or a torsion spring, preferentially hold fingers 110B and 112B as shown. However, when ring 30B is positioned at the cartridge unload station 300, as shown in Figure 1, the fingers pass by solenoids 126,128 mounted on the stationary frame (Figure 12). Such solenoids include arms 130 which are in position to pass outwardly beyond ends 122, without disturbing them, but also to press outwardly against ends 122, in the direction of arrows 132, forcing the fingers to pivot, in the direction of arrows 134, to the phantom positions. This in turn causes the fingers to release the cartridges C, which then fall out of position in the ring. A trap door in station 300 (described hereafter) allows the falling cartridge to be completely released from its track housing.

Alternatively (not shown), paths 22 and 24 can be defined by circular tracks on which a train of members 62 is caused to ride. An electric motor suitably connected to the analyzer controls can drive such a train on such a track.

To load and unload cartridge with respect to each of paths 22 and 24, load and unload station 300 is provided (as shown in Figures 1 and 13). Station 300 comprises a housing 302 which sits on the housing of the two paths (Figure 13). An opening 304 and 306 is formed in the top surface of housing 302 (Figure 1) each positioned to be aligned with an aperture of the ring below it, when the ring is appropriately centered. Positioned below openings 304 and 306 (Figure 13), is a horizontal wall 310 with an aperture 312 which can be made to be in alignment with cut-out portion 60 of the ring, and which is aligned with opening 304 or 306. An annular shoulder 314 rises from aperture 312, on which a bar code reader 316 is mounted. The bar code reader can be conventional, and is positioned to scan a bar code on an incoming cartridge as shown.

Aperture 312 is normally closed by a door 320 operated automatically by a motor 322 and any suitable linkage 324 between door 320 and its motor. When an empty portion 60 in ring 32 is positioned in place as shown, with a cartridge C' in position on door 320, motor 322 is actuated to open the door. Cartridge C' falls into place into ring 32, as supported by the structure described for Figures 6 to 12. Ring 32 can then be advanced to move a cartridge into place at the transfer device 400. Spent cartridges, on the other hand, are removed by using door 326, described hereinafter.

To transfer a test element from a stack of elements E or E' in a cartridge C, device 400 is used, as shown in Figures 1, 8 and 14. Referring especially to Figure 14, such a device features a housing 352, and movable within a slot 350 in housing, a pusher blade 414. Any kind of pusher blade is useful, but the following is preferred: All of blade 414 is flexible, except for the front end 412, which is rendered rigid by one or more ribs 416 which extend along the length of the blade. The flexible portion of the blade features notches or a rack 426 which mate with a pinion gear 432 driven in portion 430 of housing 352. As a result, the flexible portion of blade 414 can bend through the curve of slot 350, caused by housing 352 to have a radius of curvature R. This in turn minimizes the lateral space occupied by device 400, as shown in Figure 1.

Blade 414 follows path 198 above the stacks of elements E or E' presented by cartridges C or C'. These are in turn alternatively moved up into the path 198, as indicated by arrows 470 and 472, or down, depending on which type of element is desired. The cartridge in turn is selected by rotating the holding rings (not shown in Figure 14) about incubator 500 and stationary surface 510.

Referring again to Figure 8, it is desirable that the atmospheres for rings 30 and 32 be controlled, and further, that they be controlled separately. For example, relative humidity can be controlled by any mechanism. For most of station 20, this is easily done by wall 28' being a complete barrier between the two paths 22 and 24. However, to keep the atmospheres different, intermixing of atmospheres needs to be prevented when each path necessarily intersects the blade path 198. Thus, it is desirable that a seal be maintained at the top, in conjunction with the blade passage, to ensure that there is minimal air mixing between the two paths 22 and 24. A preferred embodiment to achieve these results is as follows:

The top portion 201 of the housing accommodates the passage of blade 414 and a test element dealt off the top of a stack from ring 30 or 32. More specifically, top portion 201 has a blade entrance port 202 and an exit port 204 fixed in the housing, and floating members 210, 212. The floating members are displacably seated on annular valve seats 214 disposed around an opening 216 which will allow a cartridge to be raised up into the path 198 to be engaged at groove 217 by blade 414. Members 210 and 212 are each biased downwardly against seats 214 by an expansion spring 218. Because each member 210 or 212 serves as a substitute blade guide if its cartridge is not raised up, a groove 220 horizontally extends through the member. Because each member is pushed up out of blade path 198 if its cartridge is raised up, a space 228 is formed in portion 200 to accommodate member 210 or 212 when spring 218 is compressed. (Movement upward of a stack elements E by rod 234 also causes cartridge C to be raised up against member 210 and 212.)

Although not essential, member 210 or 212 can be constructed so that top portion 230 thereof, from which boss 231 extends to receive spring 218, can be easily removed from bottom portion 232 (not shown), so that any test element jams can be readily fixed.

Elements E or E' are transferred by device 400 to support surface 510, as shown in Figures 1 and 8, where a dispensing probe 550 (Figure 8) is used to dispense a patient sample onto the test element so transferred. Any such probe 550 can be used, including those automated probes conventionally used on analyzers available under the trademark "Ektachem E-700" Analyzer from Eastman Kodak Company.

The manner in which the sample is obtained by the sample-dispensing probe is not critical to this invention. The sample dispensing probe preferably includes pressurizing means, not shown, which can be integral with the rest of the analyzer described herein, or it can be provided by a separate stand-alone sample supply station which is coordinated to act in conjunction with this analyzer. For example, the sample-dispensing means and the mechanisms for loading it, described in EP-A-0356250 entitled "Analyzers Using Linear Sample Trays with Random Access" can be used. In any event, any convenient apparatus for moving the probe from the sample location exterior of analyzer 20, to station A, as indicated by arrow 560 in Figure 1, can be used.

A dispenser (not shown) similar to 550 is used to dispense reference liquid onto a PM element, at the same location at station A as is used by probe 550. Such reference liquid dispenser is preferably conventional.

The probe first moves horizontally, as shown by arrow 560 in Figure 1, from another portion of the analyzer handling patient samples (not shown), or from a different apparatus which supplies such samples to position "A". The probe then moves downward, as indicated by arrow 562 in Figure 8, to be in position to dispense the sample.

Thereafter, blade 414 from device 400 pushes the test element further into incubator 500, as indicated by arrow 570 in Figure 1.

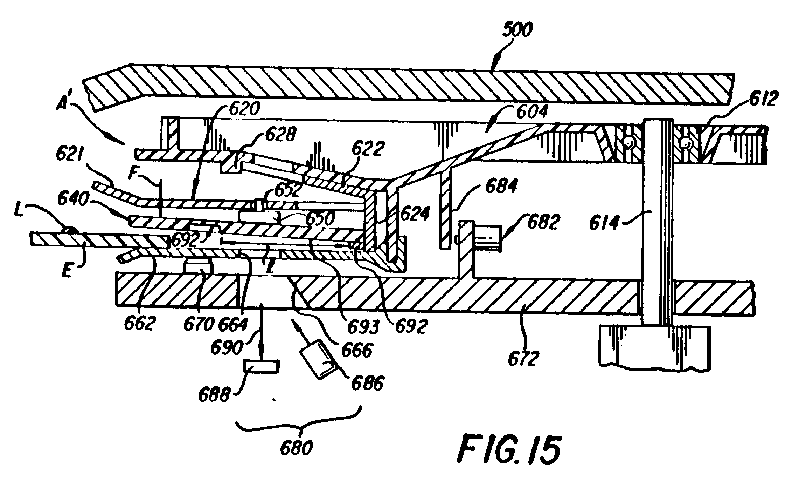

Turning next to incubator 500, it is preferably a rotating incubator with stations around its circumference each for holding a test element. Any such incubator can be used, there being a variety which are known in the art. Most preferably, it comprises, as shown in Figure 15, an incubator of the type described in commonly-owned US Patent Application Serial No. 346,205 filed on May 2, 1989 entitled "Universal Evaporation Cover". More specifically, incubator 500 preferably has a rotor 604 journaled at 612 on a shaft 614. Appropriate heating elements (not shown) are dispersed throughout incubator 500. Of the plurality of stations formed in the rotor, station A' is depicted. A spring 620 is captured at the station and is biased to press down on a cover 640 with a force F. Such a spring comprises a cover-engaging leg 621, a bias leg 622, and a vertical leg 624, leg 622 being captured behind a ridge 628 of rotor 604. A boss 650 can be mounted on the top of cover 640 to releasably engage an aperture 652 in leg 621. Vertical leg 624 rests on a support plate 662 which is apertured at 664 for the reading of colorimetric or rate-type test elements. Plate 662 is also the support of the test elements E and E', at the other stations. The undersurface of plate 662 is caused to ride over three spaced-apart buttons 670, in stationary surface 672, apertured at 666. The buttons are positioned to carefully control the height of the test element vis-a-vis a reflectometer 680, positioned to read a test element through apertures 664 and 666. Sensor 682 and flag 684 can be used to accurately sense when the stations are in their correct positions, e.g., for reading. As is conventional, reflectometer 680 preferably comprises a light source 686 and a detecting station 688 which senses the reflected light, indicated by arrow 690, from element E'.

In order for single incubator 500 to be acceptable for all three kinds of test elements (CM, rate and PM), incubator 500 is preferably modified in several ways to accommodate all three. For example, one modification is at the read station so that the reflectometer is mounted to read rate and CM test elements while still on the incubator. Because each station at the incubator may have any one of the three types of test elements present, portions such as the evaporation cover at each station are modified so as to be effective regardless of the type of test element present.

Because some CM elements may involve end-point chemistries sensitive to light, preferably a chopper (not shown) is disposed between light source 686 and each aperture 666 to allow the light beam to strike a CM type element at any station only when it is at end-point.

Regarding evaporation cover 640, as shown in Figure 15, this cover is preferably grooved at 692 to provide clearance for liquid drops L on a PM type test element (element E which is shown), while at the same time providing for a sufficient length ℓto rear edge 692 which will ensure that non-grooved surface portion 693 seals over the liquid-bearing portion of a CM or rate-type test element. (Because no liquid protrudes from such a CM or rate-type element, no clearance groove is needed). Most preferably, surface portion 693 and indeed all of cover 640 is manufactured from a non-porous material such as polyethylene.

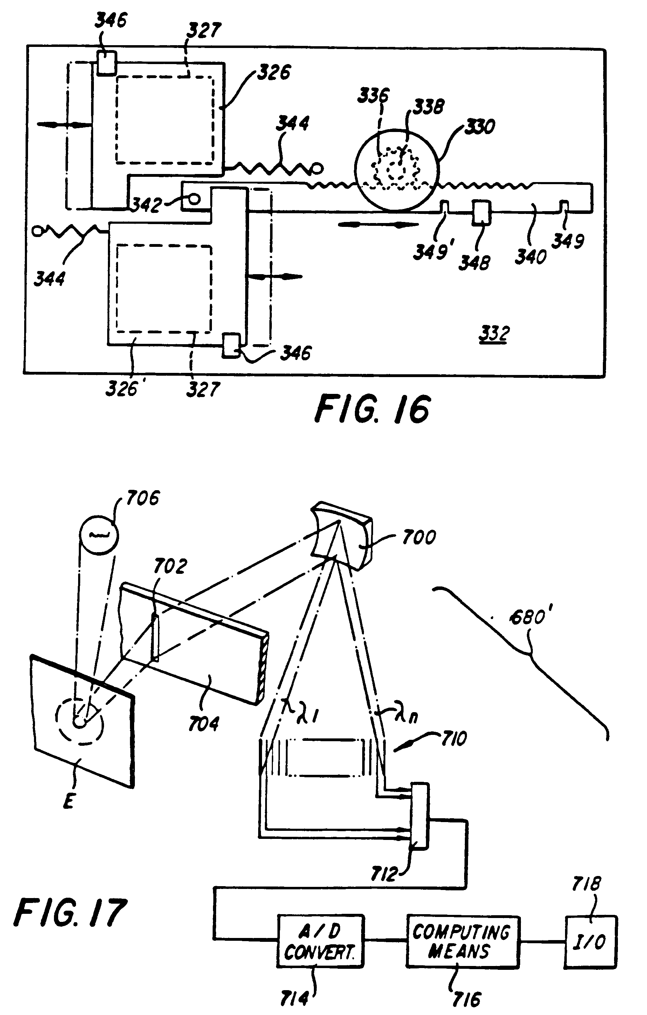

Any suitable reflectometer 680 can be used. Highly useful is a diffraction grating reflectometer 680', as shown in Figure 17. Such a reflectometer features a conventional holographic diffraction grating 700 which uses a slit 702 in mask 704 to direct a line exposure of the light reflected from element E, the element E being illuminated by a light source 706. (Fiber optics, not shown, can be used to carry the light images.) Grating 700 in turn breaks the image into a continuous spectrum of wavelengths λᵢ=λ₁ to λₙ, which fall as a linear array on an image plane 710. A suitable array of imaging detectors 712 is used to detect the array of wavelengths, wherein each element of the detector 712 detects only one of the wavelengths λᵢ, and the entire image within the wavelength. The signal from detector 712 is processed by an A/D converter 714 and sent via computing means 716 to a suitable I/O unit 718, such as a CRT display. Further details on diffraction grating reflectometers can be found in US-A-4544271.

Not all test elements are read by reflectometer 680. Specifically, PM type test elements require an electrometer 360, as shown in Figures 1 and 2. Such an electrometer is conventional, and uses two electrical probes (not shown) which are pushed down into contact with a PM test element moved out of the incubator, in the direction of arrow 362, onto support surface 510, by a conventional pusher blade 363 - see Figure 2.

After a PM test element is read, it is moved to an aperture 364 in support surface 510, where it falls out of the analyzer. A similar dump aperture 366 is provided for spent CM or rate test elements. Those are pushed from the incubator, in the direction of arrow 368, to aperture 366 by a conventional pusher blade 369.

The final station of analyzer 20 to be encountered by a cartridge, during its residence in the analyzer, is the discharge portion of station 300 (see Figures 13 and 16). This portion features a door 326, 326' for each of the two paths 22 and 24, as is especially shown in Figure 16. Each such door opens and closes aperture 327, and is independently operable by a motor 330 (Figure 13). Alternatively, they could be operated together even though it is unlikely both would be needed to discharge a spent cartridge at the same time.

A useful construction of doors 326 and 326', as shown in Figure 16, includes the following: Floor member 332 of housing 302 hangs a motor 330 from its undersurface (Figure 13) which operates a pinion gear 336 off its drive shaft 338 (Figure 16). Gear 336 in turn operates a rack 340 which has a post 342 at one end. Post 342 bears on a portion of door 326 or 326' to push such door to operate against tension spring 344 which biases its respective door closed. Sensors 346 sense the position of door 326 or 326', and sensor 348 senses a flag 349 or 349' in rack 340. In this fashion, a single motor can independently operate two independent doors for the discharge of cartridges.

## Claims

1. An analyzer for assaying for analytes of a body liquid on dried test elements, the analyzer including:-
means (C) for temporarily storing a stack of dried test elements (E, E'), the test elements (E, E') in any one stack being for the same assay and are selected from any one of potentiometric, colorimetric or rate-type test elements;
an incubator (500) for incubating test elements (E, E') taken from a stack; and
detection means (360) for detecting a change in a test element (E, E') after incubation in the incubator (500);
characterized in that the analyzer further includes at least one generally circular horizontal path (22, 24) centered generally on the incubator (500) and surrounding said incubator (500);
moving means (30, 32, 38, 40) for moving the stack-storing means (C) around each path (22, 24);
holding means (62) for temporarily holding the stack-storing means (C) on the moving means (30, 32, 38, 40), the holding means (62) being movable between at least two positions; and
transfer means (400) for transferring a test element (E, E') to the incubator (500) from any stack-storing means (C) held on a circular path (22, 24).

2. An analyzer according to claim 1, wherein the moving means comprises first moving means (30) and second moving means (32), the holding means (61, 62, 63) including a holding member (62) mounted on the first moving means (30) which is operable for moving between a first position in which the stack-storing means (C) is held on the path (22, 24) and a second position in which the stack-storing means (C) is released from the path (22, 24), and further including a third moving means (86; 110, 112; 110B, 112B) operable to cause the holding member (62) to move between the first and second positions.

3. An analyzer according to claim 2, wherein the holding means (61, 62, 63) include biasing means (90) for biasing the holding member (62) into the first position, the third moving means (86) being capable of overriding the biasing means (90) to move the holding member (62) to the second position.

4. An analyzer according to any one of claims 1 to 3, wherein each path (22, 24) comprises a ring (30, 32) having a support surface for supporting the stack-storing means (C), cut-out portions (60) being formed in the support surface, each of which is of sufficient in size to accommodate a stack-storing means (C).

5. An analyzer according to claim 4, wherein the stack-storing means comprise a cartridge and the holding member comprises a frame (62) including a cartridge-support shoulder (82) disposed at the same level as the support surface, and mounting means (74, 76) for slidably mounting the frame (62) on the support surface and partially within the cut-out portions (60) so as to move between the first position and the second position.

6. An analyzer according to claim 4 or 5, further including a second generally circular horizontal path (24) centered on the incubator (500) and spaced from the at least one path (22), and further moving means (32) for moving the stack-storing means (C) around the second path.

7. An analyzer according to claim 5 or 6, further including discharge means (326, 327) associated with each of the paths (22, 24) for discharging a stack-storing means (C) which previously contained a stack from the paths (22, 24) when the stack-storing means (C) is moved by the third moving means (86).

8. An analyzer according to claim 7, wherein the discharge means comprise a door (326, 326'), an exit port (327), and door-moving means (330) for slidably moving the door to cover or uncover the exit port.

9. An analyzer according to claim 8, wherein the door-moving means comprise a single motor connected to actuate independently the door for each of the paths.

10. An analyzer according to claim 1, wherein the third moving means comprise sliding means (86) provided for slidably moving the holding means between two positions on a radius of the circular path (22, 24), one of the positions being one in which a stack-storing means (C) is allowed to fall, and the other being one in which the stack-storing means (C) is supported.

11. An analyzer according to claim 10, wherein the holding means comprise a frame member (62) including first supporting means (82) which fits under the stack-storing means (C) to support it, and the moving means include second supporting means (63) opposite to the first means (82) and fitting under a stack-storing means (C) for supporting it, the sliding means (86) being effective to move the first and second supporting means far enough apart in one position to cause a stack-storing means (C) to fall between them.

12. An analyzer according to claim 1, wherein the moving means comprise a generally flat annular ring (30, 32) having a plurality of spaced-apart cut-out portions (60) formed therein, each cut-out portion having a size and shape to accommodate a stack-storing means (C), a portion of the ring at one edge of the cut-out shape being provided with a first pair of inwardly projecting shoulders (63), and a motor (38, 40) operable to rotate the ring around the circular path (22, 24).

13. An analyzer according to any one of the preceding claims, further including depositing means (550) for depositing a patient liquid onto a test element removed by the transfer means (400) from the stack-storing means (C), the depositing means being positionable adjacent to the incubator (500) and the transfer means (400).

14. A method for supplying a test element (E, E') to an incubator (500), each test element (E, E') being selected from any one of three different types of test elements, the method comprising the steps of:-
supplying slide-like test elements as a plurality of stacks, the test elements of each stack being identical within the stack,
mounting the stacks on at least one generally circular path (22, 24) surrounding a central incubator (500),
moving the stacks around the path until a selected stack reaches a predetermined position,
and transferring a test element (E, E') from the stack at the predetermined position to the incubator (500).

15. A method according to claim 14, wherein each stack is held in a cartridge (C), and further including the step of discarding a cartridge (C) from the path when all the elements (E, E') from the stack have been transferred.

16. A method according to claim 15, wherein the discarding step comprises the step of dropping the cartridge away from the path (22, 24).

17. A method according to any one of claims 14 to 16, wherein the transferring step comprises pushing a test element (E, E') off the top of the stack into the incubator (500).

18. A method according to any one of claims 14 to 16, wherein the transferring step comprises first transferring a test element to a position (A) outside the incubator (500), dispensing a sample liquid onto the transferred test element at the outside position, and thereafter transferring the dispensed sample and test element from the outside position into the incubator.

19. Apparatus for temporarily supporting a stack of planar slide-like elements (E, E'), in a cartridge (C) held in a predetermined vertical direction, the apparatus comprising
means (62) for temporarily holding a cartridge of elements,
means (30, 32, 38, 40) for moving the holding means along a generally circular path (22, 24) around a center,
characterized in that the apparatus includes means (86, 90) for slidably moving the holding means between two positions on a radius of the circular path, one of the positions being one which allows a cartridge to fall, and the other of the positions being one which holds up a cartridge.

20. Apparatus according to claim 19, wherein the cartridge-holding means (62) comprise opposite shoulders (63, 82) configured to fit under a portion of a cartridge (C) and the slidable moving means is constructed to move the shoulders (63, 82) together and apart.

21. Apparatus according to claim 20, wherein the slidable moving means comprise biasing means (90) for biasing the opposite shoulders (63, 82) together to occupy the other position, and means (86) for overcoming the biasing means (90) to move the opposite shoulders (63, 82) apart into the one position.

22. Apparatus according to claim 20 or 21, wherein the moving means comprise a generally flat annular ring (30, 32) having a plurality of spaced-apart cut-out portions (60) therein each having a size and shape to accommodate a cartridge (C), a portion of the ring (30, 32) at one edge of the cut-out shape being provided with a pair of inwardly projecting shoulders (63), and a motor (38, 40) operable to rotate the ring (30, 32) around the circular path (22, 24),
and the holding means comprise a frame (62) on the ring (30, 32) with a second pair of shoulders (82) projecting towards the first pair.

## Patentansprüche

1. Analysegerät zum Bestimmen von Analyten in Körperflüssigkeit mittels trägergebundener Reagenzien, bestehend aus
einem Träger (C) zur vorübergehenden Lagerung eines Stapels von die trägergebundenen Reagenzien bildenden Testelementen (E, E'), wobei die jeweils in einem Stapel vorliegenden Testelemente (E, E') für einen und denselben Test vorgesehen sind und aus potentiometrischen kolorimetrischen oder kinetischen Testelementen bestehen können,
einem Inkubator (500) zur Inkubation der einem Stapel entnommenen Testelemente (E, E'), und
einem Detektor (360) zur Feststellung einer Veränderung in den Testelementen (E, E') nach einer Inkubation im Inkubator (500),
**dadurch gekennzeichnet,** daß in dem Analysegerät mindestens eine allgemein kreisrunde waagerechte Bahn (22, 24) vorgesehen ist, die allgemein um den Mittelpunkt des Inkubators (500) zentriert ist und den Inkubator (500) umgibt,
Mittel (30, 32, 38, 40) vorgesehen sind, die den Stapelträger (C) um jede der Bahnen (22, 24) bewegen,
eine Halteeinrichtung (62) vorgesehen ist, die den Stapelträger (C) vorübergehend auf den Mitteln (30, 32, 38, 40) festhält und die zwischen mindestens zwei Stellungen bewegbar ist, und
eine Übertragungseinrichtung (400) vorgesehen ist, die ein Testelement (E, E') von jeweils einem der auf einer der kreisrunden Bahnen (22, 24) festgehaltenen Stapelträger (C) in den Inkubator (500) überführt.

2. Analysegerät nach Anspruch 1, dadurch gekennzeichnet, daß die Bewegungsmittel aus einer ersten Bewegungseinrichtung (30) und aus einer zweiten Bewegungseinrichtung (32) bestehen, daß die Halteeinrichtung (61, 62, 63) ein Halteelement (62) enthält, das auf der ersten Bewegungseinrichtung (30) gelagert ist und zwischen einer ersten Stellung, in der der Stapelträger (C) auf der Bahn (22, 24) festgehalten ist, und einer zweiten Stellung, in der der Stapelträger (C) nicht mehr auf der Bahn (22, 24) festgehalten ist, bewegbar ist, und daß eine dritte Bewegungseinrichtung (86; 110, 112; 110B, 112B) vorgesehen ist, bei deren Betätigung das Halteelement (62) zwischen seiner ersten und seiner zweiten Stellung bewegbar ist.

3. Analysegerät nach Anspruch 2, dadurch gekennzeichnet, daß die Halteeinrichtung (61, 62, 63) eine Vorspanneinrichtung (90) umfaßt, die das Halteelement (62) in seine erste Stellung vorspannt, und daß die dritte Bewegungseinrichtung (86) die Vorspanneinrichtung (90) unwirksam macht, um das Halteelement (62) in seine zweite Stellung zu bewegen.

4. Analysegerät nach einem der Ansprüche 1 bis 3, dadurch gekennzeichnet, daß jede Bahn (22, 24) einen Ring (30, 32) mit einer Lagerfläche umfaßt, auf der der Stapelträger (C) gelagert ist und in der Ausnehmungen (60) ausgebildet sind, die jeweils eine zur Aufnahme eines Stapelträgers (C) ausreichende Größe besitzen.

5. Analysegerät nach Anspruch 4, dadurch gekennzeichnet, daß der Stapelträger eine Kassette umfaßt und das Halteelement einen Rahmen (62) mit einer zur Lagerung der Kassette dienenden Schulter (82) aufweist, die sich auf der gleichen Höhe wie die Lagerfläche befindet, und daß Lagermittel (74, 76) vorgesehen sind, die den Rahmen (62) auf der Lagerfläche und teilweise in den Ausnehmungen (60) derart verschiebbar lagern, daß er zwischen der ersten Stellung und der zweiten Stellung bewegbar ist.

6. Analysegerät nach Anspruch 4 oder 5, dadurch gekennzeichnet, daß eine zweite allgemein kreisrunde, waagerechte Bahn (24) vorgesehen ist, die um den Inkubator (500) zentriert und im Abstand von mindestens der einen Bahn (22) angeordnet ist, und daß eine weitere Bewegungseinrichtung (32) den Stapelträger (C) um diese zweite Bahn bewegt.

7. Analysegerät nach Anspruch 5 oder 6, dadurch gekennzeichnet, daß es Auswerfmittel (326, 327) enthält, die jeweils einer der Bahnen (22, 24) zugeordnet sind und zum Auswerfen eines vorher mit einem Stapel bestückten Stapelträgers (C) aus den Bahnen (22, 24) dienen, wenn der Stapelträger (C) durch die dritte Bewegungseinrichtung (86) bewegt wird.

8. Analysegerät nach Anspruch 7, dadurch gekennzeichnet, daß die Auswerfmittel ein Verschlußelement (326, 326'), eine Auslaßöffnung (327) sowie eine Verschlußbetätigungseinrichtung (330) umfassen, die das Verschlußelement zum Verschließen oder Freigeben der Auslaßöffnung verschiebt.

9. Analysegerät nach Anspruch 8, dadurch gekennzeichnet, daß die Verschlußbetätigungseinrichtung aus einem einzigen Motor besteht, der so angeschlossen ist, daß er das Verschlußelement für jede der Bahnen getrennt betätigt.

10. Analysegerät nach Anspruch 1, dadurch gekennzeichnet, daß die dritte Bewegungseinrichtung aus einem Schiebeelement (86) besteht, das die Halteeinrichtung auf einem Radius der kreisrunden Bahn (22, 24) zwischen zwei Stellungen verschiebt, wobei die eine dieser Stellungen ein Herunterfallen eines Stapelträgers (C) ermöglicht, während er in der anderen der beiden Stellungen festgehalten ist.

11. Analysegerät nach Anspruch 10, dadurch gekennzeichnet, daß die Halteeinrichtung ein Rahmenteil (62) umfaßt, das eine erste Lagereinrichtung (82) enthält, die unter den Stapelträger (C) paßt und diesen lagert, und die Bewegungsmittel eine zweite gegenüber der ersten Lagereinrichtung (82) angeordnete Lagereinrichtung (63) umfassen, die unter einen Stapelträger (C) paßt und diesen lagert, wobei das Schiebeelement (86) die erste und die zweite Lagereinrichtung in der einen Stellung so weit auseinander bewegt, daß ein Stapelträger (C) zwischen ihnen hindurchfällt.

12. Analysegerät nach Anspruch 1, dadurch gekennzeichnet, daß die Bewegungsmittel einen im allgemeinen flachen, kreisförmigen Ring (30, 32) umfassen, in dem eine Vielzahl von im Abstand voneinander angeordneten Ausnehmungen (60) ausgebildet ist, die jeweils so bemessen und geformt sind, daß sie einen Stapelträger (C) aufnehmen, und daß ein Teil des Rings an einer Kante der Ausnehmung mit zwei nach innen vorspringenden Schultern (63) versehen ist und der Ring durch einen Motor (38, 40) angetrieben um die kreisförmige Bahn (22, 24) drehbar ist.

13. Analysegerät nach einem der vorhergehenden Ansprüche, dadurch gekennzeichnet, daß eine Applikatoreinrichtung (550) vorgesehen ist, die eine Patientenprobe auf ein von der Übertragungseinrichtung (400) aus dem Stapelträger (C) entnommenes Testelement überträgt und die benachbart dem Inkubator (500) und der Übertragungseinrichtung (400) positionierbar ist.

14. Verfahren zum Überführen von trägergebundene Reagenzien bildenden Testelementen (E, E') in einen Inkubator (500), wobei es sich jeweils um eines von drei verschiedenen Arten von Testelementen handeln kann, dadurch gekennzeichnet, daß
plättchenförmige Testelemente in Form einer Vielzahl von Stapeln zugeführt werden, wobei die Testelemente innerhalb eines Stapels jeweils gleich sind,
die Stapel auf mindestens eine im allgemeinen kreisrunde Bahn (22, 24) aufgebracht werden, die einen mittig angeordneten Inkubator (500) umgibt,
die Stapel um die Bahn herum bewegt werden, bis ein ausgewählter Stapel in eine vorbestimmte Position gelangt, und
ein Testelement (E, E') aus dem Stapel in der vorbestimmten Position in den Inkubator (500) überführt wird.

15. Verfahren nach Anspruch 14, dadurch gekennzeichnet, daß jeder Stapel in einer Kassette (C) enthalten ist und daß eine Kassette (C) jeweils aus der Bahn ausgeworfen wird, wenn alle Testelemente (E, E') des Stapels in den Inkubator überführt worden sind.

16. Verfahren nach Anspruch 15, dadurch gekennzeichnet, daß die Kassette dadurch ausgeworfen wird, daß sie von der Bahn (22, 24) herunterfällt.

17. Verfahren nach einem der Ansprüche 14 bis 16, dadurch gekennzeichnet, daß bei der Überführung der Testelemente jeweils ein Testelement (E, E') von der Oberseite des Stapels aus in den Inkubator (500) geschoben wird.

18. Verfahren nach einem der Ansprüche 14 bis 16, dadurch gekennzeichnet, daß bei der Überführung der Testelemente zunächst ein Testelement in eine Position (A) außerhalb des Inkubators (500) bewegt, dann eine Flüssigkeitsprobe auf das in dieser äußeren Position befindliche Testelement aufgebracht und schließlich das Testelement mit der Probe aus der äußeren Position in den Inkubator überführt wird.

19. Vorrichtung zur vorübergehenden Lagerung eines Stapels planer plättchenförmiger trägergebundene Reagenzien darstellender Testelemente (E, E') in einer in einer vorbestimmten senkrechten Richtung gehaltenen Kassette (C), mit einer Einrichtung (62), die eine Kassette mit Testelementen vorübergehend festhält, und
Mitteln (30, 32, 38, 40), die die Halteeinrichtung über eine allgemein kreisrunde Bahn (22, 24) um einen Mittelpunkt bewegen,
dadurch gekennzeichnet, daß die Vorrichtung Mittel (86, 90) enthält, die die Halteeinrichtung zwischen zwei Positionen auf einem Radius der kreisförmigen Bahn verschieben, wobei die eine der Positionen ein Herunterfallen und die andere der Positionen ein Festhalten der Kassette ermöglicht.

20. Vorrichtung nach Anspruch 19, dadurch gekennzeichnet, daß die Halteeinrichtung (62) für die Kassette einander gegenüberliegende Schultern (63, 82) umfaßt, die unter einen Teil einer Kassette (C) passen, und daß die die Halteeinrichtung verschiebenden Mittel die Schultern (63, 82) zusammen- und auseinanderbewegen.

21. Vorrichtung nach Anspruch 20, dadurch gekennzeichnet, daß die die Halteeinrichtung verschiebenden Mittel eine Vorspanneinrichtung (90) umfassen, die die einander gegenüberliegenden Schultern (63, 82) derart zueinander vorspannt, daß sie ihre Festhaltestellung einnehmen, und daß eine Einrichtung (86) vorgesehen ist, die die Vorspanneinrichtung (90) unwirksam macht, um die einander gegenüberliegenden Schultern (63, 82) auseinander in ihre Freigabestellung zu bewegen.

22. Vorrichtung nach Anspruch 20 oder 21, dadurch gekennzeichnet, daß die Bewegungsmittel einen allgemein flachen kreisrunden Ring (30, 32) umfassen, in dem eine Vielzahl von Ausnehmungen (60) im Abstand voneinander ausgebildet und so bemessen und geformt sind, daß sie eine Kassette (C) aufnehmen, wobei ein Teil des Rings (30, 32) an einer Kante der Ausnehmung mit zwei nach innen vorspringenden Schultern (63) versehen ist und der Ring (30, 32) durch einen Motor (38, 40) angetrieben um die kreisrunde Bahn (22, 24) drehbar ist, und
die Halteeinrichtung einen auf dem Ring (30, 32) befindlichen Rahmen (62) umfaßt, wobei zwei weitere Schultern (82) zu den ersten beiden Schultern hin vorspringen.

## Revendications

1. Analyseur destiné à analyser des éléments d'une humeur placée sur des éléments de test séchés, l'analyseur comprenant :
un dispositif (C) de stockage temporaire d'une pile d'éléments de test séchés (E, E'), les éléments de test (E, E' d'une pile quelconque étant destinés à une même analyse et étant sélectionnés parmi des éléments de test de type potentiométrique, colorimétrique ou de vitesse,
un incubateur (500) destiné à l'incubation d'élément de test (E, E') retiré d'une pile, et
un dispositif (360) de détection d'un changement d'un élément de test (E, E') après incubation dans l'incubateur (500),
caractérisé en ce que l'analyseur comporte en outre au moins un trajet horizontal (22, 24) de forme générale circulaire centré de façon générale sur l'incubateur (500) et entourant l'incubateur (500),
un dispositif (30, 32, 38, 40) de déplacement du dispositif (C) de stockage de pile autour de chaque trajet (22, 24),
un dispositif de maintien (62) destiné à retenir temporairement le dispositif de stockage de pile (C) sur le dispositif de déplacement (30, 32, 38, 40), le dispositif de maintien (62) étant mobile entre au moins deux positions, et
un dispositif de transfert (400) destiné à transférer un élément de test (E, E') vers l'incubateur (500) à partir d'un dispositif quelconque (C) de stockage de pile maintenu sur un trajet circulaire (22, 24).

2. Analyseur selon la revendication 1, dans lequel le dispositif de déplacement comprend un premier dispositif (30) et un second dispositif (32) de déplacement, le dispositif de maintien (61, 62, 63) comprenant un organe de maintien (62) monté sur le premier dispositif de déplacement (30) et destiné à se déplacer entre une première position dans laquelle le dispositif de stockage (C) de pile est maintenu sur le trajet (22, 24) et une seconde position dans laquelle le dispositif (C) de stockage de pile est séparé du trajet (22, 24), et comprenant en outre un troisième dispositif de déplacement (86 ; 110, 112 ; 110B, 112B) destiné à provoquer le déplacement du dispositif de maintien (62) entre la première et la seconde position.

3. Analyseur selon la revendication 2, dans lequel le dispositif de maintien (61, 62, 63) comporte un dispositif (90) de rappel de l'organe de maintien (62) vers la première position, le troisième dispositif de déplacement (86) étant capable de dépasser la force du dispositif de rappel (90) afin qu'il déplace l'organe de maintien (62) vers la seconde position.

4. Analyseur selon l'une quelconque des revendications 1 à 3, dans lequel chaque trajet (22, 24) comporte un anneau (30, 32) ayant une surface de support du dispositif (C) de stockage de pile, des parties découpées (60) étant formées à la surface de support et ayant chacune une dimension suffisante pour loger un dispositif (C) de stockage de pile.

5. Analyseur selon la revendication 4, dans lequel le dispositif de stockage de pile comporte une cartouche et l'organe de maintien comprend un cadre (62) ayant un épaulement (82) de support de cartouche placé au même niveau que la surface de support, et un dispositif (74, 76) de montage coulissant du cadre (62) sur la surface de support et partiellement dans les parties découpées (60) afin qu'il se déplace entre la première et la seconde position.

6. Analyseur selon la revendication 4 ou 5, comprenant en outre un second trajet horizontal (24) de forme générale circulaire, centré sur l'incubateur (500) et placé à distance d'au moins un trajet (22), et un dispositif supplémentaire de déplacement (32) destiné à déplacer le dispositif (C) de stockage de pile autour du second trajet.

7. Analyseur selon la revendication 5 ou 6, comprenant en outre un dispositif d'évacuation (326, 327) associé à chacun des trajets (22, 24) et destiné à l'évacuation d'un dispositif de stockage de pile (C) qui contenait auparavant une pile en dehors des trajets (22, 24) lorsque le dispositif (C) de stockage de pile est déplacé par le troisième dispositif (86) de déplacement.

8. Analyseur selon la revendication 7, dans lequel le dispositif de décharge comprend une porte (326, 326'), un orifice de sortie (327) et un dispositif (330) de déplacement de porte par coulissement afin qu'elle couvre l'orifice de sortie ou le découvre.

9. Analyseur selon la revendication 8, dans lequel le dispositif de déplacement de porte comporte un moteur unique raccordé de manière qu'il manoeuvre indépendamment la porte de chacun des trajets.

10. Analyseur selon la revendication 1, dans lequel le troisième dispositif de déplacement comprend un dispositif (86) de coulissement destiné à déplacer par coulissement le dispositif de maintien entre deux positions sur un rayon du trajet circulaire (22, 24), l'une des positions étant telle que le dispositif (C) de stockage de pile peut tomber et l'autre étant telle que ce dispositif (C) est supporté.

11. Analyseur selon la revendication 10, dans lequel le dispositif de maintien comporte un organe de cadre (62) ayant un premier dispositif de support (82) qui s'ajuste sous le dispositif (C) de stockage de pile pour le supporter, et le dispositif de déplacement comprend un second dispositif de support (63) opposé au premier dispositif (82) et se logeant sous le dispositif (C) de stockage de pile pour son support, le dispositif coulissant (86) provoquant le déplacement du premier et du second dispositif de support suffisamment loin de l'autre pour qu'un dispositif (C) de stockage de pile puisse tomber entre eux.

12. Analyseur selon la revendication 1, dans lequel le dispositif de déplacement comporte un anneau (30, 32) de forme générale plate ayant plusieurs parties découpées espacées (60), formées dans l'anneau, chaque partie découpée ayant une dimension et une configuration permettant le logement d'un dispositif (C) de stockage de pile, une partie de l'anneau placée à un bord de la forme découpée ayant une première paire d'épaulements (63) dépassant vers l'intérieur, et un moteur (38, 40) destiné à faire tourner l'anneau suivant le trajet circulaire (22, 24).

13. Analyseur selon l'une quelconque des revendications précédentes, comprenant en outre un dispositif (550) de dépôt d'un liquide d'un patient sur un élément de test retiré par le dispositif de transfert (400) du dispositif (C) de stockage de pile, le dispositif de dépôt pouvant être placé près de l'incubateur (500) et du dispositif de transfert (400).

14. Procédé de transmission d'un élément de test (E, E') à un incubateur (500), chaque élément de test (E, E') étant sélectionné parmi trois types différents d'éléments de test, le procédé comprenant les étapes suivantes :
la transmission d'éléments de test en forme de lames sous forme de plusieurs piles, les éléments de test de chaque pile étant identiques dans la pile,
le montage des piles sur au moins un trajet de forme générale circulaire (22, 24) entourant un incubateur central (500),
le déplacement des piles autour du trajet jusqu'à ce qu'une pile choisie atteigne une position prédéterminée, et
le transfert d'un élément de test (E, E') de la pile en position prédéterminée à l'incubateur (500).

15. Procédé selon la revendication 14, dans lequel chaque pile est maintenue dans une cartouche (C), et comprenant en outre une étape d'évacuation d'une cartouche (C) du trajet lorsque tous les éléments (E, E') de la pile ont été transférés.

16. Procédé selon la revendication 15, dans lequel l'étape d'évacuation comprend une étape de chute de la cartouche afin qu'elle s'éloigne du trajet (22, 24).

17. Procédé selon l'une quelconque des revendications 14 à 16, dans lequel l'étape de transfert comprend la poussée d'un element de test (E, E') à partir de la partie supérieure de la pile dans l'incubateur (500).

18. Procédé selon l'une quelconque des revendications 14 à 16, dans lequel l'étape de transfert comprend un premier transfert d'un élément de test à une position (A) qui est en dehors de l'incubateur (500), la distribution d'un échantillon liquide sur l'élément de test transféré en position externe, puis le transfert de l'échantillon distribué et de l'élément de test de la position externe jusqu'à l'intérieur de l'incubateur.

19. Appareil de support temporaire d'une pile d'éléments plans en forme de lames (E, E') dans une cartouche (C) supportée en direction verticale prédéterminée, l'appareil comprenant :
un dispositif (62) de maintien temporaire d'une cartouche d'éléments,
un dispositif (30, 32, 38, 40) de déplacement du dispositif de maintien le long d'un trajet de forme générale circulaire (22, 24) autour d'un centre,
caractérisé en ce que l'appareil comprend un dispositif (86, 90) de déplacement par coulissement du dispositif de maintien entre deux positions sur un rayon du trajet circulaire, l'une des positions étant telle qu'elle permet la chute d'une cartouche et l'autre des positions étant telle qu'une cartouche est maintenue.

20. Appareil selon la revendication 19, dans lequel le dispositif (62) de maintien de cartouche comprend des épaulements opposés (63, 82) ayant une configuration telle qu'ils s'ajustent sous une partie de la cartouche (C), et un dispositif coulissant de déplacement a une construction permettant le déplacement des épaulements (63, 82) l'un vers l'autre et à distance l'un de l'autre.

21. Appareil selon la revendication 20, dans. lequel le dispositif de déplacement coulissant comprend un dispositif (90) de rappel des épaulements opposés (63, 82) l'un vers l'autre afin qu'ils occupent l'autre position, et un dispositif (86) destiné à dépasser la force du dispositif de rappel (90) afin qu'il écarte les épaulements opposés (63, 82) vers la première position.

22. Appareil selon la revendication 20 ou 21, dans lequel le dispositif de déplacement comporte un anneau de forme générale plate (30, 32) ayant plusieurs parties découpées espacées (60) ayant chacune une dimension et une configuration permettant le logement d'une cartouche (C), une partie de l'anneau (30, 32) placée à un bord de la partie découpée ayant une paire d'épaulements (63) dépassant vers l'intérieur, et un moteur (38, 40) destiné à faire tourner l'anneau (30, 32) suivant le trajet circulaire (22, 24), et
le dispositif de maintien comporte un cadre (62) placé sur l'anneau (30, 32), une seconde paire d'épaulements (82) dépassant vers la première paire.
